# EUROPEAN PATENT APPLICATION

(11) **EP 1 982 729 A1**
(43) Date of publication of application: **22.10.2008**
(21) Application number: 07106676.5
(22) Date of filing: 20.04.2007
(51) Int. Cl.: A61K 39/385, A61K 47/48, A61P 25/30

(54) **Vaccination Regimen for B-Cell Vaccines**

(71) Applicant: Cytos Biotechnology AG, 8952 Schlieren (CH)
(72) Inventor: Bachmann, Martin F., Dr., 8472, Seuzach (CH); Maurer, Patrik, Dr., 8408 Winterthur (CH); Pfister, Thomas, Dr., 8902, Urdorf (CH); Müller, Philipp, Dr., 4304, Giebenach (CH); Renner, Wolfgang, 8802, Kilchberg (CH)
(74) Representative: Pfister-Fu, Yixin

(57) **Abstract**

This invention relates to the field of vaccination and treatment or prevention diseases. In particular this invention relates to the treatment or prevention of diseases by inducing hapten-specific antibody responses in a vaccinated subject. The invention further provides a method for prevention or treatment of a disease by inducing hapten-specific antibodies in a subject comprising administering into said subject a composition comprising a virus-like particle of an RNA bacteriophage and at least one hapten linked thereto.

## Description

### FIELD OF THE INVENTION

This invention relates to the field of vaccination and treatment or prevention diseases. In particular this invention relates to the treatment or prevention of diseases by inducing hapten-specific antibody responses in a vaccinated subject. The invention further provides a method for prevention or treatment of a disease by inducing hapten-specific antibodies in a subject comprising administering into said subject a composition comprising a virus-like particle of an RNA bacteriophage and at least one hapten linked thereto.

### PRIOR ART

The concept behind vaccination against, hapten, which includes the majority of drugs of abuse, is that the anti-drug of abuse antibodies, which do not cross the blood-brain barrier, bind the drug in the blood and thus reduce the amount and rate of drug entering the central nervous system. By lowering the rewards associated with drug use, the addicted individual should no longer be motivated to consume the drug.

Nicotine vaccines have been tested in humans. The recombinant nicotine - *Pseudomonas aeruginosa* exoprotein A conjugate NicVAX was tested in smokers which were injected four times with either 50 µg, 100 µg or 200 µg NicVAX or placebo (Hatsukami DK et al, Clin Pharmacol Ther 2005, 78:456-467) at weeks 0, 4, 8 and 26. One injection of NicVAX was not sufficient to induce anti-nicotine antibodies and nicotine-specific antibodies were detected only after the second injection. Peak anti-nicotine titers were reached only at week 10, two weeks after the third injection.

Efficacy of a nicotine vaccine in smoking cessation has been demonstrated in a double-blind, placebo-controlled phase II smoking cessation study (Comuz J et al, Presented at ASCO, May 2005; Orlando Fl, Available at http://www.asco.org/ac/1,1003,_12-002643-00_18-0034-00_19-0033424,00.asp. Accessed on November, 25, 2005). Moderate to heavy smokers of total 340 subjects were recruited and were immunized five times with 100 µg NicQb (represents for nicotine molecules linked to the virus-like particle of an RNA bacteriophage) formulated in Alum or placebo in monthly intervals. The target quit date was set to month 1 after primary immunization. Continuous abstinence between month 2 and month 6 was slightly higher in subjects treated with NicQb than for placebo-treated subjects (28% versus 21%), but the difference did not reach statistical significance. However when smokers were stratified according to their anti-nicotine antibody titers, a robust statistically significant effect of vaccination was observed. 159 smokers in the active arm were divided in three groups depending on their titers. 57% of smokers with the highest antibody responses were continuously abstinent compared to 31% of subjects who received placebo; and this effect was highly significant (p=0.004). In contrast, subjects with medium or low antibody levels did not reach significant abstinence. The increased continuous abstinence rate persisted up to one year and 42% of the subjects with high antibody titers had not relapsed compared to 21% in the placebo group (Cytos: Vaccine to treat nicotine addiction promotes 12 months continuous abstinence in subjects who achieve high antibody levels. Press Release: Company webpage, http://www.cytos.com/doc/Cytos_Press_E_051117.pdf. Accessed on November, 25, 2005). These data clearly demonstrated that a vaccine against nicotine can be efficacious for smoking cessation in humans when anti-nicotine antibody levels are high enough and the antibodies have a sufficient affinity for nicotine.

Thus, there is a need for methods to induce higher effective anti-nicotine antibody levels. This is usually achieved by increasing the vaccine doses or by addition of an adjuvant. In addition, subjects usually relapse early during smoking cessation efforts. Initial lapses usually occur a few days after the initiation of smoking cessation and most subjects relapse within the first 8 weeks. Therefore, it is desirable to induce high anti-nicotine antibody levels as early as possible.

In addition, antibodies have to be of sufficient affinity. For several haptens it has been described that 1-2 weeks after immunization only antibodies with low affinities (Kd= 1000 - 10000 nM) were induced while antibodies isolated after a few months showed affinity maturation and thus high affinities with Kd = 10 - 100 nM (Foote and Eisen, PNAS, Vol. 97(20) pp. 10679-81, 1995).

### SUMMARY OF THE INVENTION

We have surprisingly found new vaccination regimens for hapten vaccines, in particular for vaccine for the prevention and treatment of addiction to a drug of abuse. The present regimens of the invention allow higher antibody titer achievable in immunized subjects, compared to the prior art regimens with time interval between administrations of around 28 days. For example, in the regimens described in the example section, the achieved highest antibody titer doubled in the regimens of the present invention than prior art. Furthermore these inventive regimens lead to a high antibody response to be reached much earlier than prior art. Again the regimen settings in the example section showed the peak reaching time can be shortened at least by half compared to the regimen done by prior art. Despite the present accelerated vaccination regimens, we have surprisingly found that the antibody affinity from the present regimens and from the prior art regimens are both comparably high.

Thus the present invention provides a method for prevention or treatment of a disease by inducing hapten-specific antibodies in a human comprising administering into said human a composition comprising: (a) a virus-like particle of an RNA bacteriophage with at least one first attachment site; and (b) at least one hapten with at least one, preferably only one, second attachment site, wherein (a) and (b) are covalently linked through the at least one first and the at least one second attachment site, and wherein the administering into the human of the composition comprises at least a first, a second and a third administration into the human of the composition, wherein the time interval between the first administration and the second administration, and between the second administration and the third administration is at most 18 days.

In a preferred embodiment, the present invention provides a method for prevention or treatment of addiction to a drug of abuse by inducing the drug of abuse-specific antibodies in a human comprising administering into said human a composition comprising, consisting essentially of, or consisting of: (a) a virus-like particle of an RNA bacteriophage with at least one first attachment site; and (b) at least one drug of abuse with at least one, preferably only one, second attachment site, wherein (a) and (b) are covalently linked through the at least one first and the at least one second attachment site, and wherein the administering into the human of the composition comprises at least a first, a second and a third administration into the human of the composition, wherein the time interval between the first administration and the second administration, and between the second administration and the third administration is at most 18 days. In one preferred embodiment, the time interval is at most 14 days. In one further preferred embodiment, the time interval is 14 days or 7 days.

In one preferred embodiment, the method of the present invention comprises or consists of five administrations, wherein the time interval between all the administrations is 14 days or 7 days. In one preferred embodiment, the method of the present invention comprises or consists of five administrations, wherein the time interval between the first and the second, the second and the third is 7 days and the time interval between the third and the fourth and the fourth and the fifth is 14 days.

In one preferred embodiment, the composition administered by the method of the present invention further comprises an adjuvant, wherein said adjuvant is preferably an aluminum salt, preferably aluminum hydroxide.

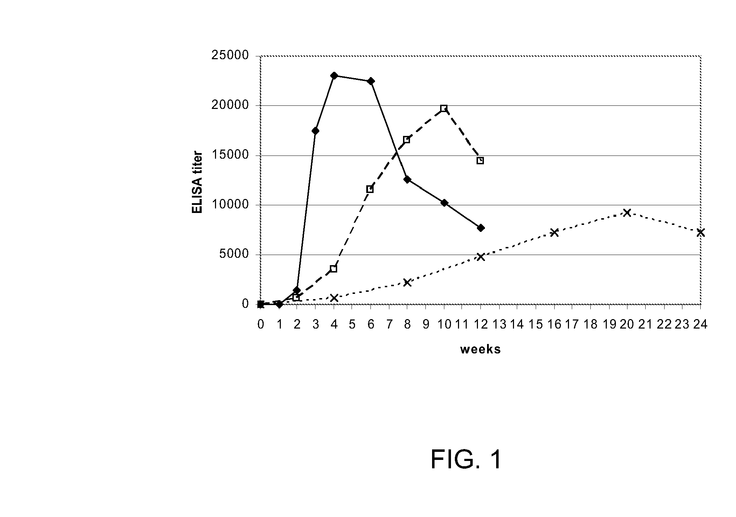

FIGURE 1: Comparison of vaccination regimens 1, 2 and 3: Subjects were immunized with 100 µg NicQb in the presence of Alum either 5 times in weekly intervals (closed diamonds, regimen 3), 5 times in biweekly intervals (open squares, regimen 2) or five times with a monthly interval (crosses, regimen 1). Nicotine-specific IgG titers were determined for all subjects by ELISA and geometric mean ELISA titers of the three groups are shown.

### DETAILED DESCRIPTION OF THE INVENTION

Unless defined otherwise, all technical and scientific terms used herein have the same meanings as commonly understood by one of ordinary skill in the art to which this invention belongs.

Adjuvant: The term "adjuvant" as used herein refers to non-specific stimulators of the immune response or substances that allow generation of a depot in the host which when combined with the composition, vaccine composition or pharmaceutical composition of the invention provides for an even more enhanced and/or prolonged immune response, preferably cytokine production. A variety of adjuvants is known in the art and useful in the invention. Preferred adjuvants are selected from the group consisting of complete or incomplete Freund's adjuvant, aluminum containing adjuvants, modified muramyldipeptide, surface active substances such as lysolecithin, pluronic polyols, polyanions, peptides, oil emulsions, keyhole limpet hemocyanins, dinitrophenol, BCG (bacille Calmette Guerin) Corynebacterium parvum, ligands of toll-like receptors (TLR) which include but are not limited to peptidoglycans, lipopolysaccharides and their derivatives, poly I:C, immunostimulatory oligonucleotides, imidazoquinolines such as resiquimod and imiquimod, flagellins, monophosphoryl lipid immunomodulator, AdjuVax 100a, QS-21, QS-18, GPI-0100, CRL1005, MF-59, OM-174, OM-197, OM-294, Virosomal adjuvant technology and any mixture thereof. A very preferred adjuvant for the purpose of the invention is aluminum containing adjuvant, preferably an aluminum salt, preferably aluminum hydroxide, preferably an aluminum containing mineral gel, most preferably alhydrogel. In a very preferred embodiment said adjuvant is alhydrogel. The term adjuvant also encompasses a mixture of any of the substances listed above. Particles of the invention, preferably VLPs, have been generally described as an adjuvant. However, the term "adjuvant", as used within the context of this application, refers to an adjuvant not being the VLP of RNA-bacteriophage used for the inventive compositions. In each case, the term adjuvant refers to an adjuvant used in addition to the VLP.

Attachment Site, First: As used herein, the term "first attachment site" refers to an element which is naturally occurring with the VLP of RNA-bacteriophage or which is artificially added to the VLP of RNA-bacteriophage, and to which the second attachment site may be linked. The first attachment site may be a protein, a polypeptide, an amino acid, a peptide, a sugar, a polynucleotide, a natural or synthetic polymer, a secondary metabolite or compound (biotin, fluorescein, retinol, digoxigenin, metal ions, phenylmethylsulfonylfluoride), or a chemically reactive group such as an amino group, a carboxyl group, a sulfhydryl group, a hydroxyl group, a guanidinyl group, histidinyl group, or a combination thereof. A preferred embodiment of a chemically reactive group being the first attachment site is the amino group of an amino acid such as lysine. The first attachment site is located, typically on the surface, and preferably on the outer surface of the VLP. Multiple first attachment sites are present on the surface, preferably on the outer surface of virus-like particle of an RNA bacteriophage, typically in a repetitive configuration.

Attachment Site, Second: As used herein, the term "second attachment site" refers to an element part of or associated with the hapten, or preferably the drug of abuse, to which the first attachment site on the surface of the VLP of RNA bacteriophage may associate. The second attachment site of the hapten, or preferably of the drug of abuse, comprises any chemical moiety, preferably a amine, an amide, a carboxyl, a sulfhydryl, hydroxyl, aldehyde, acylhalogenide, hydrazine, diazonium, or hydrazide, or further chemical moieties able to specifically react with the first attachment site. At least one, preferably only one, second attachment site is present on the hapten or preferably the drug of abuse. The term "hapten" or preferably "drug of abuse with at least one second attachment site" refers, therefore, to a hapten, or preferably a "drug of abuse", construct comprising at least the hapten, or preferably the "drug of abuse", and the second attachment site. However, in particular for a second attachment site, which is not naturally occurring within the hapten or preferably the drug of abuse, these haptens or drugs of abuse comprise a linker which associates the hapten or the drug of abuse with the second attachment site, or more preferably, already comprises or contains the second attachment site.

Coat protein: The term "coat protein" and the interchangeably used term "capsid protein" within this application, refers to a viral protein, preferably a subunit of an RNA bacteriophage, which is capable of assembling into a virus capsid or a VLP of an RNA bacteriophage.

Hapten: As used herein, the term "hapten" refers to a low-molecular weight organic compound that is not capable of eliciting an immune response by itself but will elicit an immune response once attached to a carrier molecule. Exemplary haptens used in conjugates, compositions and methods of the invention include drugs, hormones and toxins, but are not limited to these specific haptens. A majority of drug of abuse are haptens.

Linked: The term "linked" (or its noun: linkage) as used herein, refers to all possible ways, preferably chemical interactions, by which the at least one first attachment site and the at least one second attachment site are joined together. Chemical interactions include covalent and non-covalent interactions. Typical examples for non-covalent interactions are ionic interactions, hydrophobic interactions or hydrogen bonds, whereas covalent interactions are based, by way of example, on covalent bonds such as ester, ether, phosphoester, amide, peptide, carbon-phosphorus bonds, carbon-sulfur bonds such as thioether, or imide bonds. In certain preferred embodiments the first attachment site and the second attachment site are linked through at least one covalent bond. The term "linked" as used herein, however, shall not only encompass a direct linkage of the at least one first attachment site and the at least one second attachment site but also, alternatively and preferably, an indirect linkage of the at least one first attachment site and the at least one second attachment site through intermediate molecule(s), and hereby typically and preferably by using at least one, preferably one, heterobifunctional cross-linker.

Nicotine: The term "nicotine" as used in the present invention refers to nicotine, either in its enantiomerically pure (S)- or (R)-form or a mixture thereof, which could be derivatized in such manner as to contain at least one second attachment site which, then, is capable of associating with the first attachment site of the carrier either directly, or via a cross-linker. Preferably, the nicotine is derivatized in such manner as to contain only one second attachment site.

Virus-like particle of a RNA phage: As used herein, the term "virus-like particle of a RNA phage" refers to a virus-like particle comprising, or preferably consisting essentially of or consisting of coat proteins, mutants or fragments thereof, of a RNA phage. In addition, virus-like particle of a RNA phage resembling the structure of a RNA phage, being non replicative or non-infectious, and typically and preferably being non replicative and non-infectious. Typically and preferably, the term "virus-like particle of a RNA phage" should furthermore refer to a virus-like particle of a RNA phage which lacks at least one of the genes, preferably all of the genes, encoding for the replication machinery of the RNA phage, and typically and further preferably even at least one of the genes, preferably all of the genes, encoding the protein or proteins responsible for viral attachment to or entry into the host. This definition should, however, also encompass virus-like particles of RNA phages, in which the aforementioned gene or genes are still present but inactive, and, therefore, also leading to non-replicative and/or noninfectious virus-like particles of a RNA phage. Moreover, the term "virus-like particle of a RNA phage" should therefore also encompass in its broadest definition a virus particle of a RNA phage, the genome of which has been inactivated by physical or chemical or genetic methods so that the virus particle is not capable of infecting and/or replicating. Preferred VLPs derived from RNA-phages exhibit icosahedral symmetry and consist of 180 subunits. Within this present disclosure the term "subunit" and "monomer" are interexchangeably and equivalently used within this context. In this application, the term "RNA-phage" and the term "RNA-bacteriophage" are interchangeably used.

One, a, or an: when the terms "one", "a", or "an" are used in this disclosure, they mean "at least one" or "one or more" unless otherwise indicated.

The amino acid sequence identity of polypeptides can be determined conventionally using known computer programs such as the Bestfit program. When using Bestfit or any other sequence alignment program, preferably using Bestfit, to determine whether a particular sequence is, for instance, 95% identical to a reference amino acid sequence, the parameters are set such that the percentage of identity is calculated over the full length of the reference amino acid sequence and that gaps in homology of up to 5% of the total number of amino acid residues in the reference sequence are allowed. This aforementioned method in determining the percentage of identity between polypeptides is applicable to all proteins, polypeptides or a fragment thereof disclosed in this invention.

It will be understood by one of ordinary skill in the relevant arts that other suitable modifications and adaptations to the methods and applications described herein are readily apparent and may be made without departing from the scope of the invention or any embodiment thereof. In particular in this application the time interval expressed by days, such as "at most 14 days", "is 14 days", "is 7 days", is to be understood to have a derivation of plus or minus two days, preferably plus or minus one day. Other numerical values expressed herein, except the values experimentally determined within the examples, shall include the stated value and a value range of ±10% of the stated value.

In one aspect of the invention, the present invention provides a method for prevention or treatment of a disease by inducing hapten-specific antibodies in a human comprising administering into the human a composition comprising, consisting essentially of, or consisting of: (a) a virus-like particle of an RNA bacteriophage with at least one first attachment site; and (b) at least one hapten with at least one, preferably only one, second attachment site, wherein (a) and (b) are covalently linked through the at least one first and the at least one second attachment site, and wherein the administering into the human of the composition comprises at least a first, a second and a third administration into the human of the composition, wherein the time interval between the first administration and the second administration, and between the second administration and the third administration is at most 18 days.

In one aspect of the invention, the present invention provides a method for prevention or treatment of addiction to a drug of abuse by inducing the drug of abuse-specific antibodies in a human comprising administering into the human a composition comprising, consisting essentially of, or consisting of: (a) a virus-like particle of an RNA bacteriophage with at least one first attachment site; and (b) at least one drug of abuse with at least one, preferably only one, second attachment site, wherein (a) and (b) are covalently linked through the at least one first and the at least one second attachment site, and wherein the administering into the human of the composition comprises at least a first, a second and a third administration into the human of the composition, wherein the time interval between the first administration and the second administration, and between the second administration and the third administration is at most 18 days. Preferably the time interval between the first administration and the second administration, and between the second administration and the third administration is at least three days, preferably at least four days, more preferably at least five days. In one preferred embodiment, the time interval between the first administration and the second administration, and between the second administration and the third administration is at least five days and at most 18 days.

In one preferred embodiment, the time interval between the first administration and the second administration, and between the second administration and the third administration is at most 14 days, or at most 10, or preferably at most 7 days. Further preferably the time interval between the first administration and the second administration, and between the second administration and the third administration is least three days, preferably at least four days, more preferably at least five days. In one preferred embodiment, the time interval between the first administration and the second administration, and between the second administration and the third administration is at least five days and at most 14 days. In one preferred embodiment, the time interval between the first administration and the second administration, and between the second administration and the third administration is at least five days and at most 10 days. In one preferred embodiment, the time interval between the first administration and the second administration, and between the second administration and the third administration is at least five days and at most 7 days.

In one preferred embodiment, the time interval between the first administration and the second administration and the time interval between the second administration and the third administration are the same.

In a very preferred embodiment, the time interval between the first administration and the second administration, and between the second administration and the third administration is 14 days. In an alternatively very preferred embodiment, the time interval between the first administration and the second administration, and between the second administration and the third administration is 7 days.

In one preferred embodiment, the method of the present invention further comprises further administrations. In one preferred embodiment, the method of the present invention comprises or consists of a further administration, or two further administrations or three administrations.

In one preferred embodiment, the method of present invention further comprises or consists of a fourth administration. In one preferred embodiment, the time interval between the third administration and the fourth administration is equal to the time interval between the first administration and the second administration. In one preferred embodiment, the time interval between the first and the second, the second and the third and the third and the fourth are the same. In another preferred embodiment, the time interval between the third administration and the fourth administration is longer than the time interval between the first administration and the second administration and is longer than the time interval between the second administration and the third administration. In one preferred embodiment, the time interval between the third administration and the fourth administration is twice as long as the time interval between the first administration and the second administration, and is twice as long as the time interval between the second administration and the third administration. In one preferred embodiment, the time interval between the third administration and the fourth administration is at most 14 days, preferably is 14 days or preferably is 7 days.

In one preferred embodiment, the method of present invention further comprises or consists of a fifth administration. In one preferred embodiment, the time interval between the fourth administration and the fifth administration is equal to the time interval between the first administration and the second administration. In another preferred embodiment, the time interval between the fourth administration and the fifth administration is longer than the time interval between the first administration and the second administration and is longer than the time interval between the second administration and the third administration. In one preferred embodiment, the time interval between the fourth administration and the fifth administration is twice as long as the time interval between the first administration and the second administration, and is twice as long as the time interval between the second administration and the third administration. In one preferred embodiment, the time interval between the third administration and the fourth administration and the time interval between the fourth administration and the fifth administration are the same. In one preferred embodiment, the time interval between the fourth administration and the fifth administration is at most 14 days, preferably is 14 days or preferably is 7 days.

In one preferred embodiment, the time interval between the first administration and the second, the second and the third, the third and the fourth and the fourth and the fifth are all the same. In one preferred embodiment, the time interval is at most 14 days. In one further preferred embodiment, the time interval is 14 days. In another further preferred embodiment, the time interval is 7 days.

In one preferred embodiment, the time interval between the third and the fourth and between the fourth and the fifth are longer than the time interval between the first and the second and the second and the third. In one preferred embodiment, the time interval between the first administration and the second, the second and the third is 7 days and the time interval between the third and the fourth and the fourth and the fifth is 14 days. In one preferred embodiment, the time interval between the first administration and the second, the second and the third is 14 days and the time interval between the third and the fourth and the fourth and the fifth is 28 days. The advantage of after the first three initial administrations, extend the time interval of the later administrations is to obtain a prolonged high antibody titer in the subject.

In one preferred embodiment, the time interval between the first and the second, the second and the third is 7 days and between the third and the fourth, the fourth and the fifth is at least 14 days, preferably is 14 days, more preferably is 21 days and still more preferably is 28 days.

In another preferred embodiment, the time interval between the first and the second, the second and the third, the third and the fourth is 7 days and between the fourth and the fifth is at least 14 days, preferably is 14 days, more preferably is 21 days and still more preferably is 28 days.

It has been generally believed that the maturation of the antibodies, i.e., the evolvement of high affinity antibodies towards an antigen takes some time, usually several months. However we have surprising found that with our accelerated vaccination regimens, i.e., the time interval between the first administration and the second, and between the second and the third is at most 14 days, preferably the time interval is 14 days or 7 days, or with our even more preferred regimens, i.e., the time intervals between the five administrations is at most 14 days, preferably is 14 days or 7 days, not only high antibody response can be achieved within shorter time than prior art, furthermore and more importantly the obtainable or obtained antibodies have a high affinity of Kd lower than 500nM, which is comparable to prior art.

Thus in one preferred embodiment, the method of the present invention leads to the highest antibody response to be reached within 10 weeks, preferably within 4 weeks.

Furthermore in one preferred embodiment, the antibody specific for a hapten, preferably for a drug of abuse, more preferably for a nicotine molecule, obtainable or obtained from the method of the present invention, has an affinity of which Kd is lower than 500nM, preferably lower than 150nM, more preferably lower than 100nM, more preferably lower than 50nM, more preferably lower than 10nM, more preferably lower than 1nM.

In one preferred embodiment, the RNA bacteriophage is selected from the group consisting of: a) bacteriophage Qβ; b) bacteriophage R17; c) bacteriophage fr; d) bacteriophage GA; e) bacteriophage SP; f) bacteriophage MS2; g) bacteriophage M11; h) bacteriophage MX1; i) bacteriophage NL95; k) bacteriophage f2; 1) bacteriophage PP7 and m) bacteriophage AP205.

In one preferred embodiment, the virus-like particle of an RNA bacteriophage comprises, or alternatively consists essentially of, or consists of, coat proteins, recombinant coat proteins, mutants or fragments thereof, of an RNA bacteriophage. The term "fragment of a coat protein", as used herein, is defined as a polypeptide, which is of at least 70%, preferably at least 80%, more preferably at least 90%, even more preferably at least 95% the length of the wild-type coat protein and which preferably retains the capability of forming VLP of an RNA bacteriophage. Preferably the fragment is obtained by at least one internal deletion, at least one truncation or at least one combination thereof. The term "fragment of a coat protein" shall further encompass polypeptide, which has at least 80%, preferably 90%, even more preferably 95% amino acid sequence identity with the "fragment of a coat protein", as defined above and which is preferably capable of assembling into a virus-like particle of an RNA bacteriophage.

The term "mutant coat protein" or the term "mutant of a coat protein", as interchangeably used in this invention, refers to a polypeptide having an amino acid sequence derived from the coat protein, wherein the amino acid sequence is at least 80%, preferably at least 85%, 90%, 95%, 97%, or 99% identical to the coat protein and preferably retains the ability to assemble into a VLP of an RNA bacteriophage.

In one preferred embodiment of the invention, the composition comprises coat protein, mutants or fragments thereof, of RNA phages, wherein the coat protein has amino acid sequence selected from the group consisting of: (a) SEQ ID NO:1; referring to Qβ CP; (b) a mixture of SEQ ID NO:1 and SEQ ID NO:2 (referring to Qβ Al protein); (c) SEQ ID NO:3; (d) SEQ ID NO:4; (e) SEQ ID NO:5; (f) SEQ ID NO:6, (g) a mixture of SEQ ID NO:6 and SEQ ID NO:7; (h) SEQ ID NO:8; (i) SEQ ID NO:9; (j) SEQ ID NO:10; (k) SEQ ID NO:11; (1) SEQ ID NO:12; (m) SEQ ID NO:13; and (n) SEQ ID NO:14.

In one preferred embodiment, the RNA bacteriophage is Qβ. Particular example 18 of WO 02/056905 gave detailed description of preparation of VLP particles from Qβ.

Qβ mutant coat protein, of which exposed lysine residues are replaced by arginines can be used for the present invention. In a preferred embodiment, Qβ mutant coat protein is selected from the group consisting of: a) Qβ-240 (SEQ ID NO:15, Lys13-Arg of SEQ ID NO: 1) b) Qβ-243 (SEQ ID NO:16, Asn10-Lys of SEQ ID NO:1); c) Qβ-250 (SEQ ID NO:17, Lys2-Arg of SEQ ID NO:1) d) Qβ-251 (SEQ ID NO:18, Lys16-Arg of SEQ ID NO:1); and e) Qβ-259" (SEQ ID NO:19, Lys2-Arg, Lys16-Arg of SEQ ID NO:1).

In one preferred embodiment, the RNA bacteriophage is AP205.

In one preferred embodiment, the first attachment site comprises, or preferably is, an amino group, preferably the amino group of a lysine residue.

In one preferred embodiment, the drug of abuse is selected from the group consisting of:
(a) codeine; (b) fentanyl; (c) heroin; (d) morphine; (e) amphetamine; (f) cocaine; (g) methylenedioxymethamphetamine; (h) methamphetamine; (i) methylphenidate; (j) nicotine; (k) cotinine; (1) nornicotine; (m) PCP; (n) LSD; (o) mescaline; (p) psilocybin; (q) tetrahydrocannabinol; (r) diazepam; (s) desipramine; (t) imipramine; (u) nortriptyline; and (v) the amitriptyline class of drugs.

In one preferred embodiment, the drug of abuse is nicotine. The totality of the covalently bound nicotine molecules are either present in the same absolute configuration, i.e. all nicotine molecules have the (R)-configuration or all nicotine molecules have the naturally occurring (*S*)-configuration, or they are present in any mixture thereof. Preferably, the nicotine molecules are covalently bound such as about an equal mixture or an equal mixture of both the (*R*)-configuration and the naturally occurring (*S*)-configuration is present. In a very preferred embodiment, the nicotine-VLP conjugate comprised by the inventive formulations is obtainable or obtained by using a racemic mixture of nicotine molecules or nicotine derivatives, typically and preferably by using a racemic mixture of nicotine molecules or nicotine derivatives comprising the nicotine molecules with a linking sequence covalently bound thereto, for the coupling reaction to the VLP of RNA bacteriophage.

In one preferred embodiment, the drug of abuse is nicotine metabolites including nornicotine and cotinine. Therapy for nicotine addiction may also target nicotine metabolites including nornicotine and cotinine, both of which have longer half lives than nicotine, have pharmacologic and neuropharmacologic affects similar to nicotine and may be addictive. US6932971, in particular from column 42 to 43, describes different sites of linkage and means of linkage of the nicotine or nicotine metabolites to the protein carrier.

In one preferred embodiment, the drug of abuse is cocaine, for example succinylated norcocaine. US6932971, in particular from column 43 to 45 till the second paragraph, describes different sites of linkage and means of linkage of the cocaine and related drugs to the protein carrier.

In one preferred embodiment, the drug of abuse is heroine.

Suitable second attachment site, which naturally occurs with or is derivable from the hapten, preferably the drug of abuse, are known to a skilled person. US 6932971 describes, in particular, from column 37 to column 41, the nature, location and generation of second attachment site and these teachings are incorporated herein by way of reference. Chemical derivatization of a hapten, preferably a drug of abuse, leads to said haptens, or preferably said drug of abuse, comprises a linker which associates the hapten, or preferably drug of abuse, with the second attachment site, or more preferably, already comprises or contains the second attachment site.

In one very preferred embodiment, the composition administered by the present method comprises (a) a virus-like particle of an RNA bacteriophage Qβ and (b) at least one nicotine molecule, wherein the at least one nicotine molecule is covalently bound to the virus-like particle by a linking sequence, wherein the linking sequence consists of A-CH₂OCO(CH₂)₂CO-B, and wherein A represents said nicotine molecule and wherein B represents said virus-like particle of RNA bacteriophage Qβ, and wherein said linking sequence is covalently bound to the 3' position of said nicotine molecule.

In one preferred embodiment, the composition administered by the method of the invention further comprises an adjuvant, wherein preferably is aluminum containing adjuvant, preferably an aluminum salt, preferably aluminium hydroxide, preferably an aluminum containing mineral gel, most preferably alhydrogel. In one preferred embodiment, the composition administered by the method of the invention comprises from 1mg to 2mg, preferably from 1.2mg to 1.7mg, more preferably from 1.3mg to 1.5mg of aluminium salt, preferably aluminium hydroxide.

In one preferred embodiment, during each administration substantially the same dose of said composition is administered.

In one preferred embodiment, during each administration a dose of at least 50 µg of the composition, preferably at least 100 µg, or preferably at least 200 µg or at least 300 µg is administered. The dose of the administered composition shall not preferably exceed 500 µg, preferably not exceeds 400 µg. In one preferred embodiment, during each administration about 100 µg of the composition is administered in a human by the method of the present invention.

The compositions of the present invention may be administered by various methods known in the art, but will normally be administered by injection, infusion, inhalation, oral administration, or other suitable physical methods. The compositions may alternatively be administered intramuscularly, intravenously, transmucosally, transdermally or subcutaneously. In one very preferred embodiment, the administration of the composition is administered subcutaneously. Components of compositions for administration include sterile aqueous (e.g., physiological saline) or non-aqueous solutions and suspensions. Examples of non-aqueous solvents are propylene glycol, polyethylene glycol, vegetable oils such as olive oil, and injectable organic esters such as ethyl oleate. Carriers or occlusive dressings can be used to increase skin permeability and enhance antigen absorption.

### EXAMPLES

### EXAMPLE 1

"NicQβ" - The term "NicQβ", as used herein should refer to at least one nicotine-VLP conjugate comprising (a) a virus-like particle of RNA bacteriophage Qβ; and (b) at least one nicotine molecule, wherein the nicotine molecule is covalently bound to VLP of Qβ by a linking sequence, wherein said linking sequence consists of A-CH₂OCO(CH₂)₂CO-B, and wherein A represents said nicotine molecule and wherein B represents the VLP of Qβ, and wherein the linking sequence is covalently bound to the 3' position of the nicotine molecule. NicQβ was produced as described in EXAMPLE 1 of US 6'932'971. NicQβ drug substance was thawed at room temperature (regimen 1) or reconstituted with sterile water from a freeze-dried powder followed by the addition of an adjuvant Alhydrogel™ containing aluminum hydroxide [Al(OH)₃].

The final vaccine composition ready for administration, designated as "100µg-dose", containing 100µg of NicQβ and 1.3mg of aluminum hydroxide.

### EXAMPLE 2

Regimen 1: Subject received five doses prepared as described in Example 1. The time interval between the administrations was 28 days
Regimen 2: Substantially the same as Regimen 1 with the exception that the time interval between the administrations was 14 days.
Regimen 3: Substantially the same as Regimen 1 with the exception that the time interval between the administrations was 7 days.

Figure 1: Comparison of weekly, biweekly and monthly vaccination regimen:

Blood samples were collected at the time points as indicated in Figure 1 for the measurement of anti-nicotine antibody titers to evaluate the immunogenicity of the particular dosing regimen. Anti-nicotine IgG antibody titer was determined by ELISA using well plates coated with RNAse-nicotine conjugate. The geometric mean values of antibody titers were shown in Figure 1. The antibody binding affinity to nicotine was determined by equilibrium dialysis.

While with regimen 1 the highest antibody titer was reached at week 20, the peak was reached at week 10 and week 4 for regimen 2 and 3 respectively.

The highest antibody titers are 9000, 20000, and 23000 for regimen 1, 2 and 3 respectively. Thus the highest antibody titer achieved by regimen 3 and regimen 2 are about two times higher than that achieved by regimen 1.

At week 8 tested, all three regimens led to nicotine-specific antibodies with comparable affinity, which is around 50-100nM.

## Claims

1. A method for prevention or treatment of addiction to a drug of abuse by inducing the drug of abuse-specific antibodies in a human comprising administering into said human a composition comprising:
(a) a virus-like particle of an RNA bacteriophage with at least one first attachment site,
(b) at least one drug of abuse with at least one second attachment site,
wherein (a) and (b) are covalently linked through said at least one first and said at least one second attachment site,
and wherein said administering into said human of said composition comprises at least a first, a second and a third administration into said human of said composition, wherein the time interval between said first administration and said second administration, and between said second administration and said third administration is at most 18 days.

2. The method of claim 1, wherein said time interval between said first administration and said second administration, and between said second administration and said third administration is at most 14 days.

3. The method of claim 1 or claim 2, wherein said time interval between said first administration and said second administration, and between said second administration and said third administration is 7 days.

4. The method of any one of the preceding claims further comprising a fourth administration.

5. The method of claim 4 further comprising a fifth administration.

6. The method any of claim 5, wherein the time interval between the first administration and the second, the second and the third, the third and the fourth and the fourth and the fifth is 7 days.

7. The method of any of the claims 1-6, wherein the time interval between the third and the fourth and between the fourth and the fifth are longer than the time interval between the first and the second and is longer than the time interval between the second and the third.

8. The method of claim 7, wherein the time interval between the first administration and the second, the second and the third is 7 days and the time interval between the third and the fourth and the fourth and the fifth is 28 days.

9. The method of any of the preceding claims, wherein said drug of abuse is nicotine.

10. The method of any one of the preceding claims, wherein said disease is nicotine addiction.


SEQUENCE LISTING

<110>  Cytos Biotechnology AG
       Bachmann, Martin F
       Maurer, Patrik
       Pfister, Thomas
       Müller, Philipp
       Wolfgang, Renner

<120>  Vaccination Regimen for B-Cell Vaccines

<130>  P1072EP00

<160>  19

<170>  PatentIn version 3.3

<210>  1
<211>  132
<212>  PRT
<213>  Bacteriophage Q-beta

<400>  1

Ala Lys Leu Glu Thr Val Thr Leu Gly Asn Ile Gly Lys Asp Gly Lys
1               5                   10                  15

Gln Thr Leu Val Leu Asn Pro Arg Gly Val Asn Pro Thr Asn Gly Val
            20                  25                  30

Ala Ser Leu Ser Gln Ala Gly Ala Val Pro Ala Leu Glu Lys Arg Val
        35                  40                  45

Thr Val Ser Val Ser Gln Pro Ser Arg Asn Arg Lys Asn Tyr Lys Val
    50                  55                  60

Gln Val Lys Ile Gln Asn Pro Thr Ala Cys Thr Ala Asn Gly Ser Cys
65                  70                  75                  80

Asp Pro Ser Val Thr Arg Gln Ala Tyr Ala Asp Val Thr Phe Ser Phe
                85                  90                  95

Thr Gln Tyr Ser Thr Asp Glu Glu Arg Ala Phe Val Arg Thr Glu Leu
            100                 105                 110

Ala Ala Leu Leu Ala Ser Pro Leu Leu Ile Asp Ala Ile Asp Gln Leu
        115                 120                 125

Asn Pro Ala Tyr
    130

<210>  2
<211>  329

<212>  PRT
<213>  Bacteriophage Q-beta

<400>  2

Met Ala Lys Leu Glu Thr Val Thr Leu Gly Asn Ile Gly Lys Asp Gly
1               5                   10                  15

Lys Gln Thr Leu Val Leu Asn Pro Arg Gly Val Asn Pro Thr Asn Gly
            20                  25                  30

Val Ala Ser Leu Ser Gln Ala Gly Ala Val Pro Ala Leu Glu Lys Arg
        35                  40                  45

Val Thr Val Ser Val Ser Gln Pro Ser Arg Asn Arg Lys Asn Tyr Lys
    50                  55                  60

Val Gln Val Lys Ile Gln Asn Pro Thr Ala Cys Thr Ala Asn Gly Ser
65                  70                  75                  80

Cys Asp Pro Ser Val Thr Arg Gln Ala Tyr Ala Asp Val Thr Phe Ser
                85                  90                  95

Phe Thr Gln Tyr Ser Thr Asp Glu Glu Arg Ala Phe Val Arg Thr Glu
            100                 105                 110

Leu Ala Ala Leu Leu Ala Ser Pro Leu Leu Ile Asp Ala Ile Asp Gln
        115                 120                 125

Leu Asn Pro Ala Tyr Trp Thr Leu Leu Ile Ala Gly Gly Gly Ser Gly
    130                 135                 140

Ser Lys Pro Asp Pro Val Ile Pro Asp Pro Pro Ile Asp Pro Pro Pro
145                 150                 155                 160

Gly Thr Gly Lys Tyr Thr Cys Pro Phe Ala Ile Trp Ser Leu Glu Glu
                165                 170                 175

Val Tyr Glu Pro Pro Thr Lys Asn Arg Pro Trp Pro Ile Tyr Asn Ala
            180                 185                 190

Val Glu Leu Gln Pro Arg Glu Phe Asp Val Ala Leu Lys Asp Leu Leu
        195                 200                 205

Gly Asn Thr Lys Trp Arg Asp Trp Asp Ser Arg Leu Ser Tyr Thr Thr
    210                 215                 220

Phe Arg Gly Cys Arg Gly Asn Gly Tyr Ile Asp Leu Asp Ala Thr Tyr
225                 230                 235                 240

Leu Ala Thr Asp Gln Ala Met Arg Asp Gln Lys Tyr Asp Ile Arg Glu
                245                 250                 255

Gly Lys Lys Pro Gly Ala Phe Gly Asn Ile Glu Arg Phe Ile Tyr Leu
            260                 265                 270

Lys Ser Ile Asn Ala Tyr Cys Ser Leu Ser Asp Ile Ala Ala Tyr His
        275                 280                 285

Ala Asp Gly Val Ile Val Gly Phe Trp Arg Asp Pro Ser Ser Gly Gly
    290                 295                 300

Ala Ile Pro Phe Asp Phe Thr Lys Phe Asp Lys Thr Lys Cys Pro Ile
305                 310                 315                 320

Gln Ala Val Ile Val Val Pro Arg Ala
                325

<210>  3
<211>  129
<212>  PRT
<213>  Bacteriophage R17

<400>  3

Ala Ser Asn Phe Thr Gln Phe Val Leu Val Asn Asp Gly Gly Thr Gly
1               5                   10                  15

Asn Val Thr Val Ala Pro Ser Asn Phe Ala Asn Gly Val Ala Glu Trp
            20                  25                  30

Ile Ser Ser Asn Ser Arg Ser Gln Ala Tyr Lys Val Thr Cys Ser Val
        35                  40                  45

Arg Gln Ser Ser Ala Gln Asn Arg Lys Tyr Thr Ile Lys Val Glu Val
    50                  55                  60

Pro Lys Val Ala Thr Gln Thr Val Gly Gly Val Glu Leu Pro Val Ala
65                  70                  75                  80

Ala Trp Arg Ser Tyr Leu Asn Met Glu Leu Thr Ile Pro Ile Phe Ala
                85                  90                  95

Thr Asn Ser Asp Cys Glu Leu Ile Val Lys Ala Met Gln Gly Leu Leu
            100                 105                 110

Lys Asp Gly Asn Pro Ile Pro Ser Ala Ile Ala Ala Asn Ser Gly Ile
        115                 120                 125

Tyr

<210> 4
<211> 130
<212> PRT
<213> Bacteriophage fr

<400> 4

Met Ala Ser Asn Phe Glu Glu Phe Val Leu Val Asp Asn Gly Gly Thr
1               5                   10                  15

Gly Asp Val Lys Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Asn Asn Arg Lys Tyr Thr Val Lys Val Glu
    50                  55                  60

Val Pro Lys Val Ala Thr Gln Val Gln Gly Gly Val Glu Leu Pro Val
65                  70                  75                  80

Ala Ala Trp Arg Ser Tyr Met Asn Met Glu Leu Thr Ile Pro Val Phe
                85                  90                  95

Ala Thr Asn Asp Asp Cys Ala Leu Ile Val Lys Ala Leu Gln Gly Thr
            100                 105                 110

Phe Lys Thr Gly Asn Pro Ile Ala Thr Ala Ile Ala Ala Asn Ser Gly
        115                 120                 125

Ile Tyr
    130

<210> 5
<211> 130
<212> PRT
<213> Bacteriophage GA

<400> 5

Met Ala Thr Leu Arg Ser Phe Val Leu Val Asp Asn Gly Gly Thr Gly
1               5                   10                  15

Asn Val Thr Val Val Pro Val Ser Asn Ala Asn Gly Val Ala Glu Trp
            20                  25                  30

Leu Ser Asn Asn Ser Arg Ser Gln Ala Tyr Arg Val Thr Ala Ser Tyr
        35                  40                  45

Arg Ala Ser Gly Ala Asp Lys Arg Lys Tyr Ala Ile Lys Leu Glu Val
    50                  55                  60

Pro Lys Ile Val Thr Gln Val Val Asn Gly Val Glu Leu Pro Gly Ser
65                  70                  75                  80

Ala Trp Lys Ala Tyr Ala Ser Ile Asp Leu Thr Ile Pro Ile Phe Ala
                85                  90                  95

Ala Thr Asp Asp Val Thr Val Ile Ser Lys Ser Leu Ala Gly Leu Phe
            100                 105                 110

Lys Val Gly Asn Pro Ile Ala Glu Ala Ile Ser Ser Gln Ser Gly Phe
        115                 120                 125

Tyr Ala
    130

<210> 6
<211> 132
<212> PRT
<213> Bacteriophage SP

<400> 6

Met Ala Lys Leu Asn Gln Val Thr Leu Ser Lys Ile Gly Lys Asn Gly
1               5                   10                  15

Asp Gln Thr Leu Thr Leu Thr Pro Arg Gly Val Asn Pro Thr Asn Gly
            20                  25                  30

Val Ala Ser Leu Ser Glu Ala Gly Ala Val Pro Ala Leu Glu Lys Arg
        35                  40                  45

Val Thr Val Ser Val Ala Gln Pro Ser Arg Asn Arg Lys Asn Phe Lys
    50                  55                  60

Val Gln Ile Lys Leu Gln Asn Pro Thr Ala Cys Thr Arg Asp Ala Cys
65                  70                  75                  80

Asp Pro Ser Val Thr Arg Ser Ala Phe Ala Asp Val Thr Leu Ser Phe
                85                  90                  95

Thr Ser Tyr Ser Thr Asp Glu Glu Arg Ala Leu Ile Arg Thr Glu Leu
            100                 105                 110

Ala Ala Leu Leu Ala Asp Pro Leu Ile Val Asp Ala Ile Asp Asn Leu
        115                 120                 125

Asn Pro Ala Tyr
    130

<210>  7
<211>  329
<212>  PRT
<213>  Bacteriophage SP

<400>  7

Ala Lys Leu Asn Gln Val Thr Leu Ser Lys Ile Gly Lys Asn Gly Asp
1               5                   10                  15

Gln Thr Leu Thr Leu Thr Pro Arg Gly Val Asn Pro Thr Asn Gly Val
            20                  25                  30

Ala Ser Leu Ser Glu Ala Gly Ala Val Pro Ala Leu Glu Lys Arg Val
        35                  40                  45

Thr Val Ser Val Ala Gln Pro Ser Arg Asn Arg Lys Asn Phe Lys Val
    50                  55                  60

Gln Ile Lys Leu Gln Asn Pro Thr Ala Cys Thr Arg Asp Ala Cys Asp
65                  70                  75                  80

Pro Ser Val Thr Arg Ser Ala Phe Ala Asp Val Thr Leu Ser Phe Thr
                85                  90                  95

Ser Tyr Ser Thr Asp Glu Glu Arg Ala Leu Ile Arg Thr Glu Leu Ala
            100                 105                 110

Ala Leu Leu Ala Asp Pro Leu Ile Val Asp Ala Ile Asp Asn Leu Asn
        115                 120                 125

Pro Ala Tyr Trp Ala Ala Leu Leu Val Ala Ser Ser Gly Gly Gly Asp
    130                 135                 140

Asn Pro Ser Asp Pro Asp Val Pro Val Val Pro Asp Val Lys Pro Pro
145                 150                 155                 160

Asp Gly Thr Gly Arg Tyr Lys Cys Pro Phe Ala Cys Tyr Arg Leu Gly
                165                 170                 175

Ser Ile Tyr Glu Val Gly Lys Glu Gly Ser Pro Asp Ile Tyr Glu Arg
            180                 185                 190

Gly Asp Glu Val Ser Val Thr Phe Asp Tyr Ala Leu Glu Asp Phe Leu
        195                 200                 205

Gly Asn Thr Asn Trp Arg Asn Trp Asp Gln Arg Leu Ser Asp Tyr Asp
    210                 215                 220

Ile Ala Asn Arg Arg Arg Cys Arg Gly Asn Gly Tyr Ile Asp Leu Asp
225                 230                 235                 240

Ala Thr Ala Met Gln Ser Asp Asp Phe Val Leu Ser Gly Arg Tyr Gly
                245                 250                 255

Val Arg Lys Val Lys Phe Pro Gly Ala Phe Gly Ser Ile Lys Tyr Leu
            260                 265                 270

Leu Asn Ile Gln Gly Asp Ala Trp Leu Asp Leu Ser Glu Val Thr Ala
        275                 280                 285

Tyr Arg Ser Tyr Gly Met Val Ile Gly Phe Trp Thr Asp Ser Lys Ser
    290                 295                 300

Pro Gln Leu Pro Thr Asp Phe Thr Gln Phe Asn Ser Ala Asn Cys Pro
305                 310                 315                 320

Val Gln Thr Val Ile Ile Ile Pro Ser
                325

<210>  8
<211>  130
<212>  PRT
<213>  Bacteriophage MS2

<400>  8

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Met Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  9
<211>  133
<212>  PRT
<213>  Bacteriophage M11

<400>  9

Met Ala Lys Leu Gln Ala Ile Thr Leu Ser Gly Ile Gly Lys Lys Gly
1               5                   10                  15

Asp Val Thr Leu Asp Leu Asn Pro Arg Gly Val Asn Pro Thr Asn Gly
            20                  25                  30

Val Ala Ala Leu Ser Glu Ala Gly Ala Val Pro Ala Leu Glu Lys Arg
        35                  40                  45

Val Thr Ile Ser Val Ser Gln Pro Ser Arg Asn Arg Lys Asn Tyr Lys
    50                  55                  60

Val Gln Val Lys Ile Gln Asn Pro Thr Ser Cys Thr Ala Ser Gly Thr
65                  70                  75                  80

Cys Asp Pro Ser Val Thr Arg Ser Ala Tyr Ser Asp Val Thr Phe Ser
                85                  90                  95

Phe Thr Gln Tyr Ser Thr Val Glu Glu Arg Ala Leu Val Arg Thr Glu
            100                 105                 110

Leu Gln Ala Leu Leu Ala Asp Pro Met Leu Val Asn Ala Ile Asp Asn
        115                 120                 125

Leu Asn Pro Ala Tyr
    130

<210>  10
<211>  133
<212>  PRT

<213>  Bacteriophage MX1

<400>  10

Met Ala Lys Leu Gln Ala Ile Thr Leu Ser Gly Ile Gly Lys Asn Gly
1               5                   10                  15

Asp Val Thr Leu Asn Leu Asn Pro Arg Gly Val Asn Pro Thr Asn Gly
            20                  25                  30

Val Ala Ala Leu Ser Glu Ala Gly Ala Val Pro Ala Leu Glu Lys Arg
        35                  40                  45

Val Thr Ile Ser Val Ser Gln Pro Ser Arg Asn Arg Lys Asn Tyr Lys
    50                  55                  60

Val Gln Val Lys Ile Gln Asn Pro Thr Ser Cys Thr Ala Ser Gly Thr
65                  70                  75                  80

Cys Asp Pro Ser Val Thr Arg Ser Ala Tyr Ala Asp Val Thr Phe Ser
                85                  90                  95

Phe Thr Gln Tyr Ser Thr Asp Glu Glu Arg Ala Leu Val Arg Thr Glu
            100                 105                 110

Leu Lys Ala Leu Leu Ala Asp Pro Met Leu Ile Asp Ala Ile Asp Asn
        115                 120                 125

Leu Asn Pro Ala Tyr
    130


<210>  11
<211>  330
<212>  PRT
<213>  Bacteriophage NL95

<400>  11

Met Ala Lys Leu Asn Lys Val Thr Leu Thr Gly Ile Gly Lys Ala Gly
1               5                   10                  15

Asn Gln Thr Leu Thr Leu Thr Pro Arg Gly Val Asn Pro Thr Asn Gly
            20                  25                  30

Val Ala Ser Leu Ser Glu Ala Gly Ala Val Pro Ala Leu Glu Lys Arg
        35                  40                  45

Val Thr Val Ser Val Ala Gln Pro Ser Arg Asn Arg Lys Asn Tyr Lys
    50                  55                  60

Val Gln Ile Lys Leu Gln Asn Pro Thr Ala Cys Thr Lys Asp Ala Cys
65                  70                  75                  80

Asp Pro Ser Val Thr Arg Ser Gly Ser Arg Asp Val Thr Leu Ser Phe
                85                  90                  95

Thr Ser Tyr Ser Thr Glu Arg Glu Arg Ala Leu Ile Arg Thr Glu Leu
            100                 105                 110

Ala Ala Leu Leu Lys Asp Asp Leu Ile Val Asp Ala Ile Asp Asn Leu
        115                 120                 125

Asn Pro Ala Tyr Trp Ala Ala Leu Leu Ala Ala Ser Pro Gly Gly Gly
    130                 135                 140

Asn Asn Pro Tyr Pro Gly Val Pro Asp Ser Pro Asn Val Lys Pro Pro
145                 150                 155                 160

Gly Gly Thr Gly Thr Tyr Arg Cys Pro Phe Ala Cys Tyr Arg Arg Gly
                165                 170                 175

Glu Leu Ile Thr Glu Ala Lys Asp Gly Ala Cys Ala Leu Tyr Ala Cys
            180                 185                 190

Gly Ser Glu Ala Leu Val Glu Phe Glu Tyr Ala Leu Glu Asp Phe Leu
        195                 200                 205

Gly Asn Glu Phe Trp Arg Asn Trp Asp Gly Arg Leu Ser Lys Tyr Asp
    210                 215                 220

Ile Glu Thr His Arg Arg Cys Arg Gly Asn Gly Tyr Val Asp Leu Asp
225                 230                 235                 240

Ala Ser Val Met Gln Ser Asp Glu Tyr Val Leu Ser Gly Ala Tyr Asp
                245                 250                 255

Val Val Lys Met Gln Pro Pro Gly Thr Phe Asp Ser Pro Arg Tyr Tyr
            260                 265                 270

Leu His Leu Met Asp Gly Ile Tyr Val Asp Leu Ala Glu Val Thr Ala
        275                 280                 285

Tyr Arg Ser Tyr Gly Met Val Ile Gly Phe Trp Thr Asp Ser Lys Ser
    290                 295                 300

Pro Gln Leu Pro Thr Asp Phe Thr Arg Phe Asn Arg His Asn Cys Pro
305                 310                 315                 320

Val Gln Thr Val Ile Val Ile Pro Ser Leu
                325                 330

<210>  12
<211>  129
<212>  PRT
<213>  Bacteriophage f2

<400>  12

Ala Ser Asn Phe Thr Gln Phe Val Leu Val Asn Asp Gly Gly Thr Gly
1               5                   10                  15

Asn Val Thr Val Ala Pro Ser Asn Phe Ala Asn Gly Val Ala Glu Trp
            20                  25                  30

Ile Ser Ser Asn Ser Arg Ser Gln Ala Tyr Lys Val Thr Cys Ser Val
        35                  40                  45

Arg Gln Ser Ser Ala Gln Asn Arg Lys Tyr Thr Ile Lys Val Glu Val
    50                  55                  60

Pro Lys Val Ala Thr Gln Thr Val Gly Gly Val Glu Leu Pro Val Ala
65                  70                  75                  80

Ala Trp Arg Ser Tyr Leu Asn Leu Glu Leu Thr Ile Pro Ile Phe Ala
                85                  90                  95

Thr Asn Ser Asp Cys Glu Leu Ile Val Lys Ala Met Gln Gly Leu Leu
            100                 105                 110

Lys Asp Gly Asn Pro Ile Pro Ser Ala Ile Ala Ala Asn Ser Gly Ile
        115                 120                 125

Tyr

<210>  13
<211>  128
<212>  PRT
<213>  Bacteriophage PP7

<400>  13

Met Ser Lys Thr Ile Val Leu Ser Val Gly Glu Ala Thr Arg Thr Leu
1               5                   10                  15

Thr Glu Ile Gln Ser Thr Ala Asp Arg Gln Ile Phe Glu Glu Lys Val
            20                  25                  30

Gly Pro Leu Val Gly Arg Leu Arg Leu Thr Ala Ser Leu Arg Gln Asn
        35                  40                  45

Gly Ala Lys Thr Ala Tyr Arg Val Asn Leu Lys Leu Asp Gln Ala Asp
    50                  55                  60

Val Val Asp Cys Ser Thr Ser Val Cys Gly Glu Leu Pro Lys Val Arg
65                  70                  75                  80

Tyr Thr Gln Val Trp Ser His Asp Val Thr Ile Val Ala Asn Ser Thr
                85                  90                  95

Glu Ala Ser Arg Lys Ser Leu Tyr Asp Leu Thr Lys Ser Leu Val Ala
            100                 105                 110

Thr Ser Gln Val Glu Asp Leu Val Val Asn Leu Val Pro Leu Gly Arg
        115                 120                 125

<210>  14
<211>  131
<212>  PRT
<213>  bacteriophage AP205

<400>  14

Met Ala Asn Lys Pro Met Gln Pro Ile Thr Ser Thr Ala Asn Lys Ile
1               5                   10                  15

Val Trp Ser Asp Pro Thr Arg Leu Ser Thr Thr Phe Ser Ala Ser Leu
            20                  25                  30

Leu Arg Gln Arg Val Lys Val Gly Ile Ala Glu Leu Asn Asn Val Ser
        35                  40                  45

Gly Gln Tyr Val Ser Val Tyr Lys Arg Pro Ala Pro Lys Pro Glu Gly
    50                  55                  60

Cys Ala Asp Ala Cys Val Ile Met Pro Asn Glu Asn Gln Ser Ile Arg
65                  70                  75                  80

Thr Val Ile Ser Gly Ser Ala Glu Asn Leu Ala Thr Leu Lys Ala Glu
                85                  90                  95

Trp Glu Thr His Lys Arg Asn Val Asp Thr Leu Phe Ala Ser Gly Asn
            100                 105                 110

Ala Gly Leu Gly Phe Leu Asp Pro Thr Ala Ala Ile Val Ser Ser Asp
        115                 120                 125

Thr Thr Ala
    130

<210>  15
<211>  132
<212>  PRT
<213>  Artificial Sequence

<220>
<223>  Bacteriophage Qbeta 240 mutant

<400>  15

Ala Lys Leu Glu Thr Val Thr Leu Gly Asn Ile Gly Arg Asp Gly Lys
1               5                   10                  15

Gln Thr Leu Val Leu Asn Pro Arg Gly Val Asn Pro Thr Asn Gly Val
            20                  25                  30

Ala Ser Leu Ser Gln Ala Gly Ala Val Pro Ala Leu Glu Lys Arg Val
        35                  40                  45

Thr Val Ser Val Ser Gln Pro Ser Arg Asn Arg Lys Asn Tyr Lys Val
    50                  55                  60

Gln Val Lys Ile Gln Asn Pro Thr Ala Cys Thr Ala Asn Gly Ser Cys
65                  70                  75                  80

Asp Pro Ser Val Thr Arg Gln Lys Tyr Ala Asp Val Thr Phe Ser Phe
                85                  90                  95

Thr Gln Tyr Ser Thr Asp Glu Glu Arg Ala Phe Val Arg Thr Glu Leu
            100                 105                 110

Ala Ala Leu Leu Ala Ser Pro Leu Leu Ile Asp Ala Ile Asp Gln Leu
        115                 120                 125

Asn Pro Ala Tyr
    130

<210>  16
<211>  132
<212>  PRT
<213>  Artificial Sequence

<220>
<223>  Bacteriophage Q-beta 243 mutant

<400>  16

Ala Lys Leu Glu Thr Val Thr Leu Gly Lys Ile Gly Lys Asp Gly Lys
1               5                   10                  15

Gln Thr Leu Val Leu Asn Pro Arg Gly Val Asn Pro Thr Asn Gly Val
            20                  25                  30

Ala Ser Leu Ser Gln Ala Gly Ala Val Pro Ala Leu Glu Lys Arg Val
        35                  40                  45

Thr Val Ser Val Ser Gln Pro Ser Arg Asn Arg Lys Asn Tyr Lys Val
    50                  55                  60

Gln Val Lys Ile Gln Asn Pro Thr Ala Cys Thr Ala Asn Gly Ser Cys
65                  70                  75                  80

Asp Pro Ser Val Thr Arg Gln Lys Tyr Ala Asp Val Thr Phe Ser Phe
                85                  90                  95

Thr Gln Tyr Ser Thr Asp Glu Glu Arg Ala Phe Val Arg Thr Glu Leu
            100                 105                 110

Ala Ala Leu Leu Ala Ser Pro Leu Leu Ile Asp Ala Ile Asp Gln Leu
        115                 120                 125

Asn Pro Ala Tyr
    130

<210>  17
<211>  132
<212>  PRT
<213>  Artificial Sequence

<220>
<223>  Bacteriophage Q-beta 250 mutant

<400>  17

Ala Arg Leu Glu Thr Val Thr Leu Gly Asn Ile Gly Arg Asp Gly Lys
1               5                   10                  15

Gln Thr Leu Val Leu Asn Pro Arg Gly Val Asn Pro Thr Asn Gly Val
            20                  25                  30

Ala Ser Leu Ser Gln Ala Gly Ala Val Pro Ala Leu Glu Lys Arg Val
        35                  40                  45

Thr Val Ser Val Ser Gln Pro Ser Arg Asn Arg Lys Asn Tyr Lys Val
    50                  55                  60

Gln Val Lys Ile Gln Asn Pro Thr Ala Cys Thr Ala Asn Gly Ser Cys
65                  70                  75                  80

Asp Pro Ser Val Thr Arg Gln Lys Tyr Ala Asp Val Thr Phe Ser Phe
                85                  90                  95

Thr Gln Tyr Ser Thr Asp Glu Glu Arg Ala Phe Val Arg Thr Glu Leu
            100                 105                 110

Ala Ala Leu Leu Ala Ser Pro Leu Leu Ile Asp Ala Ile Asp Gln Leu
        115                 120                 125

Asn Pro Ala Tyr
    130

<210>  18
<211>  132
<212>  PRT
<213>  Artificial Sequence

<220>
<223>  Bacteriophage Q-beta 251 mutant

<400>  18

Ala Lys Leu Glu Thr Val Thr Leu Gly Asn Ile Gly Lys Asp Gly Arg
1               5                   10                  15

Gln Thr Leu Val Leu Asn Pro Arg Gly Val Asn Pro Thr Asn Gly Val
            20                  25                  30

Ala Ser Leu Ser Gln Ala Gly Ala Val Pro Ala Leu Glu Lys Arg Val
        35                  40                  45

Thr Val Ser Val Ser Gln Pro Ser Arg Asn Arg Lys Asn Tyr Lys Val
    50                  55                  60

Gln Val Lys Ile Gln Asn Pro Thr Ala Cys Thr Ala Asn Gly Ser Cys
65                  70                  75                  80

Asp Pro Ser Val Thr Arg Gln Lys Tyr Ala Asp Val Thr Phe Ser Phe
                85                  90                  95

Thr Gln Tyr Ser Thr Asp Glu Glu Arg Ala Phe Val Arg Thr Glu Leu
            100                 105                 110

Ala Ala Leu Leu Ala Ser Pro Leu Leu Ile Asp Ala Ile Asp Gln Leu
        115                 120                 125

Asn Pro Ala Tyr
    130

<210>  19
<211>  132
<212>  PRT
<213>  Artificial Sequence

<220>
<223>  Bacteriophage Q-beta 259 mutant

<400>  19

Ala Arg Leu Glu Thr Val Thr Leu Gly Asn Ile Gly Lys Asp Gly Arg
1               5                   10                  15

Gln Thr Leu Val Leu Asn Pro Arg Gly Val Asn Pro Thr Asn Gly Val
            20                  25                  30

Ala Ser Leu Ser Gln Ala Gly Ala Val Pro Ala Leu Glu Lys Arg Val
        35                  40                  45

Thr Val Ser Val Ser Gln Pro Ser Arg Asn Arg Lys Asn Tyr Lys Val
    50                  55                  60

Gln Val Lys Ile Gln Asn Pro Thr Ala Cys Thr Ala Asn Gly Ser Cys
65                  70                  75                  80

Asp Pro Ser Val Thr Arg Gln Lys Tyr Ala Asp Val Thr Phe Ser Phe
                85                  90                  95

Thr Gln Tyr Ser Thr Asp Glu Glu Arg Ala Phe Val Arg Thr Glu Leu
            100                 105                 110

Ala Ala Leu Leu Ala Ser Pro Leu Leu Ile Asp Ala Ile Asp Gln Leu
        115                 120                 125

Asn Pro Ala Tyr
    130

1